# EUROPEAN PATENT SPECIFICATION

(11) **EP 0 943 014 B1**
(45) Date of publication and mention of the grant of the patent: **21.02.2007**
(21) Application number: 97947569.6
(22) Date of filing: 14.11.1997
(51) Int. Cl.: C12Q 1/68

(54) **METHODS FOR DIAGNOSING GLAUCOMA AND DISCOVERING ANTI-GLAUCOMA DRUGS**
VERFAHREN FÜR DIE DIAGNOSE VON GLAUKOM UND FÜR DIE ERKENNUNG VON MEDIZINEN FÜR SEINE BEHANDLUNG
PROCEDES PERMETTANT DE DIAGNOSTIQUER LE GLAUCOME ET DE DECOUVRIR DES MEDICAMENTS ANTIGLAUCOMATEUX

(30) Priority: 05.12.1996 US 33227 P
(43) Date of publication of application: 22.09.1999
(73) Proprietor: Alcon Manufacturing, Ltd., Fort Worth, TX 76134 (US); UNIVERSITY OF NORTH TEXAS HEALTH SCIENCE CENTER, Fort Worth, TX 76107 (US)
(72) Inventor: Clark, Abbot F., Arlington, TX 76017 (US); Wordinger, Robert J., Euless, TX 76039 (US)
(74) Representative: Keller, Günter
(86) International application number: PCT/US1997/021054
(87) International publication number: WO 1998/024932

(56) References cited:
- WO-A-96/14411
- WO-A-96/33287
- C. ABBOT: "Sterpids, ocular hypertension and glaucoma" JOURNAL OF GLAUCOMA, vol. 4, 1995, pages 354-369, XP002059807 cited in the application
- HERNÁNDEZ ET AL.: "Glucocorticoid target cells in human outflow pathway: autopsy and surgical specimens" INVESTIGATIVE OPHTALMOLOGY & VISUAL SCIENCE, vol. 24, 1983, pages 1612-1616, XP002059808 cited in the application
- BAMBERGER ET AL.: "Glucocorticoid receptor beta, a potential endogenous inhibitor of glucocorticoid action in humans" JOURNAL OF CLINICAL INVESTIGATION, vol. 95, June 1995, pages 2435-2441, XP002059809 cited in the application
- OAKLEY ET AL.: "The human glucocorticoid receptor beta isoform" JOURNAL OF BIOLOGICAL CHEMISTRY, vol. 271, no. 16, April 1996, pages 9550-9559, XP002059810 cited in the application

## Description

Priority is claimed from the provisional application, U.S. Patent Application Serial No. 60/033227 filed December 5, 1996.

### Background of the Invention

Glaucoma is usually diagnosed by monitoring a patient's visual field loss, changes in the appearance of their optic disc, and their intraocular pressure. Glaucoma is currently treated using one or more of three strategies to lower the elevated intraocular pressure associated with the disease: with pharmaceuticals (such as beta-blockers, carbonic anhydrase inhibitors, and miotics), with laser trabeculoplasty, and/or with glaucoma filtration surgery. All of these therapies indirectly lower intraocular pressure but do not address the underlying disease process occurring in the trabecular meshwork. It would be advantageous to be able to diagnose glaucoma before a patient begins experiencing a loss in their visual field and deterioration of their optic disc.

There is a large body of evidence suggesting that glucocorticoids are involved in the generation of ocular hypertension and glaucoma. See Clark, A. F., *Journal of Glaucoma,* "Steroids, Ocular Hypertension, and Glaucoma," 4:354-369, 1995. Several investigators have shown that the human trabecular meshwork (TM) contains the classical glucocorticoid receptor (GRα). See Weinreb, et al., *Invest. Ophthalmol. Vis. Sci.,* "Detection of Glucocorticoid Receptors in Cultured Human Trabecular Cells," 21:3, 403-407, 1981, and Hernandez, et al., *Invest. Ophthalmol. Vis. Sci.,* "Glucocorticoid Target Cells in Human Outflow Pathway: Autopsy and Surgical Specimens," 24:1612-1616, 1983. Recently, the expression of an alternatively spliced form of the human glucocorticoid receptor (GRβ) was discovered in non-ocular tissues and cells. See Bamberger, et al., *The Journal of Clinical Investigation,* "Glucocorticoid Receptor β, a Potential Endogenous Inhibitor of Glucocorticoid Action in Humans," 95:2435-2441, 1995, and Oakley, et al., *The Journal of Biological Chemistry,* "The Human Glucocorticoid Receptor β Isoform," 271:16, 9550-9559, 1996. This alternatively spliced form of the glucocorticoid receptor (GR) is expressed as a protein which no longer binds glucocorticoids, but is able to interfere with the activated form of the normal glucocorticoid receptor and block or alter physiological functions of the glucocorticoid receptor.

### Summary of the Invention

The present invention is directed to methods for diagnosing glaucoma by testing a person for aberrant GRβ expression. Also set forth are methods for screening for therapeutic agents useful for treating glaucoma.

### Description of Preferred Embodiments

Surprisingly, it has been found that cultured human trabecular meshwork cell lines derived from glaucomatous donors express mRNA for both an alternate splice form of the human glucocorticoid receptor (GRβ), as well as the normal glucocorticoid receptor (GRα), whereas normal TM cell lines only express mRNA for GRα. It is believed that the elevated intraocular pressure associated with primary open-angle glaucoma may be due to the aberrant expression of GRβ in the trabecular meshwork. Therefore, determining that an individual abnormally expresses GRβ in their trabecular meshwork or other tissues can lead to a diagnosis of glaucoma. Also, this discovery can be used to determine whether agents have therapeutic value in treating glaucoma by determining whether they interact with GRβ or alter the expression of GRβ. This can be done using ligand binding assays or GRβ functional assays.

Diagnosing aberrant GRβ expression or defects in the GR gene which encodes GRβ can be done by using procedures well known to those skilled in the art. See Caskey, C. T., *J.A.M.A.,* "Molecular Medicine. A Spin-off From the Helix," 269:15, 1986-1992, 1993. For example, subjects could be screened for the presence of a genetic defect in GRβ by analyzing the DNA derived from peripheral blood leukocytes. Types of DNA analyses could include, but would not be limited to: restriction fragment length polymorphisms (RFLP), single-stranded conformation polymorphisms (SSCP), polymerase chain reaction (PCR), denaturing gradient gels, allele specific oligonucleotide ligation assay, and allele specific hybridization assay. In addition, trabecular meshwork, or other relevant cells from subjects could be analyzed for GRβ expression by a number of techniques such as reverse-transcription polymerase chain reaction (RT-PCR), immunoassays, GR functional assays, etc.

WO 96/14411 discloses a method for diagnosing glaucoma in a patient which comprises determining whether the amount of a trabecular meshwork induced glucocorticoid response protein present in the trabecular meshwork of an eye of a patient exceeds the amount of that trabecular meshwork induced glucocorticoid response protein present in the trabecular meshwork of an eye of an individual who is not suffering from glaucoma, wherein the detection of an excessive amount of the trabecular meshwork induced glucocorticoid response protein is indicative of Glaucoma.

## Claims

1. A method for diagnosing glaucoma which comprises detecting aberrant alternate splice form of the human glucocorticoid receptor (GRβ) expression or defects in a GR gene which encodes GRβ.

2. The method of Claim 1 wherein GR gene defects are detected by a method selected from the group of assays consisting of: restriction fragment length polymorphism (RFLP), single-stranded conformation polymorphism (SSCP), polymerase chain reaction (PCR), denaturing gradient gel, allele specific oligonucleotide ligation, and allele specific hybridization.

3. A method for diagnosing glaucoma, which comprises detecting genetic changes in the GR gene leading to altered GRβ expression.

4. A method for diagnosing glaucoma, which comprises detecting genetic changes outside the GR gene which lead to altered GRβ expression.

5. A method for determining whether an agent is useful for treating glaucoma by determining whether it interacts with GRβ or alters the expression of GRβ.

## Patentansprüche

1. Verfahren zur Diagnose eines Glaukoms, umfassend den Nachweis einer aberranten Expression einer alternativen Spleißform des menschlichen Glucocorticoidrezeptors (GRβ), oder von Defekten in einem GRβ codierenden GR-Gen.

2. Verfahren nach Anspruch 1, bei dem GR-Gendefekte durch ein Verfahren nachgewiesen werden, ausgewählt aus der Gruppe von Tests, bestehend aus: Restriktionsfragmentlänge-Polymorphismus (RFLP), Einzelstrang-Konformations-Polymorphismus (SSCP), Polymerasekettenreaktion (PCR), denaturierendem Gradientengel, allel-spezifischer Oligonucleotidligation sowie allel-spezifischer Hybridisierung.

3. Verfahren zur Diagnose eines Glaukoms, umfassend den Nachweis genetischer Veränderungen im GR-Gen, die zu einer veränderten GRβ-Expression führen.

4. Verfahren zur Diagnose eines Glaukoms, umfassend den Nachweis genetischer Veränderungen außerhalb des GR-Gens, die zu einer veränderten GRβ-Expression führen.

5. Verfahren zur Bestimmung, ob ein Mittel brauchbar zur Behandlung eines Glaukoms ist, durch Bestimmung, ob es mit GR-β interagiert oder die Expression von GRβ verändert.

## Revendications

1. Procédé pour diagnostiquer le glaucome comprenant la détection d'une autre forme épissée aberrante de l'expression du récepteur humain des glucocorticoïdes (GRβ) ou de défauts dans un gène du GR qui code pour le GRβ.

2. Procédé selon la revendication 1, dans lequel les défauts du gène du GR sont détectés par un procédé choisi dans le groupe d'analyses constitué par : le polymorphisme de longueur des fragments de restriction (RFLP), le polymorphisme de conformation simple brin (SSCP), la réaction en chaîne par polymérase (PCR), le gel en gradient dénaturant, une technique de ligature des oligonucléotides spécifiques d'allèle et une technique d'hybridation spécifique d'allèle.

3. Procédé pour diagnostiquer le glaucome, lequel comprend la détection de changements génétiques dans le gène de GR aboutissant à l'expression altérée du GRβ.

4. Procédé pour diagnostiquer le glaucome, lequel comprend la détection de changements génétiques à l'extérieur du gène de GR aboutissant à l'expression altérée du GRβ.

5. Procédé pour déterminer si un agent est utile pour traiter le glaucome en déterminant s'il interagit avec le GRβ ou s'il altère l'expression du GRβ.
